Europäisches Patentamt

European Patent Office

Office européen des brevets

(11) Publication number: **0 310 314**
**A1**

## EUROPEAN PATENT APPLICATION

(21) Application number: 88308900.5

(22) Date of filing: 23.09.88

(51) Int. Cl.⁴: **G01N 33/68**

(30) Priority: 24.09.87 GB 8722469

(43) Date of publication of application:
05.04.89 Bulletin 89/14

(84) Designated Contracting States:
AT BE CH DE ES FR GB GR IT LI LU NL SE

(71) Applicant: CAMBRIDGE LIFE SCIENCES PLC
Cambridge Science Park Milton Road
Cambridge CB4 4GN(GB)

(72) Inventor: Steward, Adrian Paul
5 Moulton Road Cottages Newmarket
Suffolk(GB)
Inventor: Blackmore, David John
18a The Crescent Littleport
Ely Cambridgeshire(GB)

(74) Representative: Lambert, Hugh Richmond et
al
D. YOUNG & CO. 10 Staple Inn
London, WC1V 7RD(GB)

(54) Protein assay device and method.

(57) An IgG dipstick or other device is disclosed for
the colourimetric detection of IgG in a sample in the
presence of other proteins. The dipstick or other
device comprises a porous ion exchange member 1,
e.g. of ion exchange filter paper, into which the IgG
containing sample is drawn by capillary attraction.
The pH of the sample is adjusted to the isoelectric
point of IgG, and due to the faster diffusion rate of
IgG at its isoelectric point as compared with other
proteins in the sample which have different isoelec-
tric points, IgG is preferentially separated from the
other proteins and its presence is detected by a
band of protein reactive dye 2 incorporated into the
porous ion exchange member at a predetermined
distance from the point of application of the protein
sample.

FIG.1a

## PROTEIN ASSAY DEVICE AND METHOD

### FIELD OF INVENTION

This invention relates to a method and device for the chromogenic detection and determination of a protein, e.g. immunoglobulin (IgG), in a sample comprising a plurality of other proteins which potentially interfere with the chromogenic detection and determination of the protein of interest, and which does not require the initial separation of the protein of interest from the interfering species. As a particular example, the invention is directed to a method for the chromogenic detection and determination of immunoglobulin (IgG) levels in samples of whole blood, blood serum, milk and colostrum.

### BACKGROUND

In the management of livestock, e.g. cattle, it is frequently necessary to monitor IgG levels in blood, blood serum, milk or colostrum. Whilst this can be done by laboratory techniques, a need exisits for a simple, rapid, inexpensive technique which can be carried out by the farmer or stockman without the need for sophisticated laboratory equipment, and which can be done on a routine basis, and with minimal skill or training.

In the field of protein detection, it is already known, for example, to measure albumin in urine samples by a dipstick technique, an indicator on the dipstick responding colorimetrically to the albumin (protein) content of the urine sample. Such known techniques are, however, unselective and measure total protein content, rather than selected protein content. A simple device, e.g. a dipstick, which can selectively determine a given protein in a mixture of proteins would therefore be desirable, not only for IgG determination, but possibly also the determination of other specific proteins.

### PRIOR ART

During the preparation of this application, the following prior art has been considered:

French Patent 2,422,699, which discloses a process wherein proteins can be separated by chromatography on an ion exchange column.

US Patent 3,438,737, which discloses a process for the chromogenic detection of proteins using acid indicators where the indicator test strips comprise a "bibulous" carrier, the prefered carrier material being filter paper. Such carriers will not fulfill the function of the present invention, that is to say simultaneous separation and detection of a given protein from a mixture of protein

GB 2,118,301, which discloses an integral multilayer element for the quantitative analysis of proteins comprising in sequence a water impermeable transparent support, a reagent layer, and a liquid sample spreading layer, the characteristic feature being a reagent layer comprising a protein adsorbent and an indicator, and a non-proteinaceous binder capable of allowing permeation of the protein into the protein adsorbent for contact with the indicator. Suggestions for use as the protein adsorbent layer include ion exchange resins, as suggested in the present invention. However, this prior art states the need for a separate diffusion controlling layer, whereas the present invention realises that proteins can be separated by passing the sample through an ion exchange resin, separation occuring by virtue of the different diffusion rates through the ion exchange resin.

Chemical Abstracts Vol. 86, 1977, No. 85771a, which discloses that proteins can be separated by passage through a chromatography column, and more significantly, the collection of the eluate and the subsequent photometric determination of IgG in the eluate by the addition of biuret.

Thus the prior art does not refer to a method or device for the chromogenic separation and detection of a protein from a sample comprising a plurality of proteins.

### SUMMARY OF INVENTION

According to this invention, the detection and determination of a specific protein is achieved by allowing the unfractionated protein sample, in admixture with an aqueous buffer solution having a pH value substantially equal to the isoelectric point of the protein of interest, to be drawn by capillary attraction into a porous member, e.g. a dipstick or filter paper, of ion exchange material through which the selected protein, being at its isoelectric point, diffuses at a greater rate than other proteins present in the sample and which, by virtue of their differing isoelectric points, are retained by the ion exchange material, the porous ion exchange member being of restricted volume, whereby there is established along the length of the member a concentration gradient of the protein of interest having a maximum at a point intermediate the ends of the member, said maximum being linearly separated from the point of maximum concentration of the other proteins in the sample, said member having an indicator dye incorporated into the ion exchange material at the point of maximum concentration of the protein of interest, and colorimetrically responsive to the concentration of the protein of interest at that point. As a result of the above method, a band of colour develops on the porous ion exchange member, e.g. dipstick, the intensity of colouration being proportional to the concentration of the protein of interest in the sample, which concentration can be determined by reference to appropriate colour charts.

## DETAILED DESCRIPTION

According to the present invention, the user is provided with a very simple and easy method of determining the concentration of a given protein in a protein containing sample, e.g. for determining the IgG concentration of a blood, milk or colostrum sample from cattle or other livestock. All the user need do is mix the sample with the buffer, which can be provided in prepackaged form, place a drop of the mixture on a piece of ion exchange material, e.g. an ion exchange filter paper, incorporating the indicator dye, or dipping an ion exchange dipstick incorporating the indicator into the sample/buffer mixture, and await the development of a band of colour on the ion exchange dipstick or filter.

Either the sample can be premixed with the buffer prior to application to the ion exchange dipstick or filter, or more simply still, a spot of blood, for example, or milk or colostrum, can be collected on the end of an ion exchange dipstick, and that end then immersed in a suitable buffer solution to cause the sample to be drawn up through the dipstick.

Whilst the method of the present invention is preferably carried out using a dipstick formed from a porous, ion-exchange paper, said dipstick having said dye incorporated or impregnated therein at a position spaced along the length of the dipstick at a predetermined distance from said end, there may be used instead a miniature ion-exchange column, for example, a compacted column of ion-exchange resin particles or powder compacted in a transparent tubular casing, said column having one end sealed if necessary by a suitably porous sealing member, and which end may be dipped into the buffer solution containing the sample thereby to permit the sample to be drawn up into the column by capillary attraction, the miniature ion-exchange column having said dye incorporated therein at a predetermined distance from said end.

In yet another technique, there may be used a filter paper constructed, for example, of ion-exchange paper, and onto which a drop of the buffer solution containing the sample is placed at a predetermined location. As a result of the radial diffusion of the sample away from the point of application the subsequent contact of the analyte of interest with a ring of dye concentrically applied to the filter paper at a predetermined radius from the point at which the sample is applied will cause a coloured ring to develop on the filter paper.

Any suitable aqueous buffer having a selected pH to match the isoelectric point of the protein of interest can be used. Preferred is an aqueous tris buffer solution.

In a second aspect of the invention there is provided a device for performing the above method, said device comprising a porous member of ion-exchange material having an end or region to which the sample optionally premixed with the buffer soltuion may be applied for transportation therethrough by capillary attraction, and incorporated or impregnated in said porous member at a predetermined distance from said end or said region of application, an indicator dye chromogenically reactive with the protein of interest.

Preferably the device takes the form of a dipstick comprising a strip of ion-exchange paper, which may be dipped into the buffered sample at one end, and impregnated with a band of dye at a predetermined distance from said end. In an alternative form, the device takes the form of a filter paper of ion-exchange material having incorporated or impregnated therein a circular band of dye having a predetermined radial spacing from a given point on the surface of the paper, and at which the sample is applied.

As will be appreciated the method and device of the invention consists essentially of a chromatographic ion-exchange column which effects the chromatographic separation of the proteins in the sample by virtue of their different rates of diffusion through the column, and specifically the greater rate of diffusion of the protein of interest through the column by virtue of its isoelectric point, and in which the column comprises a band of indicator dye at a predetermined spacing from the end of the column corresponding to the point of maximum concentration of the protein of interest in the restricted volume column.

Any suitable ion-exchange material may be used for the purpose of the present invention, both anion-exchange and cation-exchange. These may be selected according to principles well established and known in the art of protein separation by ion exchange chromatography, and dependent upon the isoelectric point of the protein of interest, and other proteins in the sample, and the salt content and pH of the buffer, all three of which factors play a part in determining the preferential diffusion through the ion exchange material of the protein of interest. In general, cellulosic ion-exchange materials are preferred, e.g. diethylaminoethyl cellulose (DEAEC), aminoethyl cellulose, carboxymethyl cellulose, benzyldiethylaminoethyl cellulose, oxycellulose, triethylaminoethyl cellulose, epichlorohydrin triethanolamine cellulose, benzoyl or naphthoyl diethylaminoethyl cellulose, polyethylenimine cellulose and others. For the specific determination of IgG in blood, milk and colostrum an anionic cellulosic ion exchange material is preferred, especially DEAEC.

As to the indicator dye, any indicator may be used which reacts chromogenically with the protein in question at its isoelectric point to produce a coloured product. For the detection and determination of immunoglobulin, the preferred indicator is 3,3,5,5,tetrachlorophenol 3,4,5,6,tetrabromo sulpho phthalein. Other suitable indicators may be selected by the person skilled in the art by reference to any standard reference work on pH indicators.

The invention is further described with reference to the accompaning drawings, in which:

Figure 1a is a face view of an immunoglobulin (or any other selected protein) dipstick according to the invention;

Figure 1b is a side view of the dipstick shown in Figure 1a;

Figure 2 is a face view of an alternative embodiment of the present invention; and

Figure 3 shows the concentration gradient of the selected protein and other proteins in the sample, which is obtained following application of the buffered protein containing sample to the end of the dipstick (Figure 1) or to the point X on the filter paper (Figure 2).

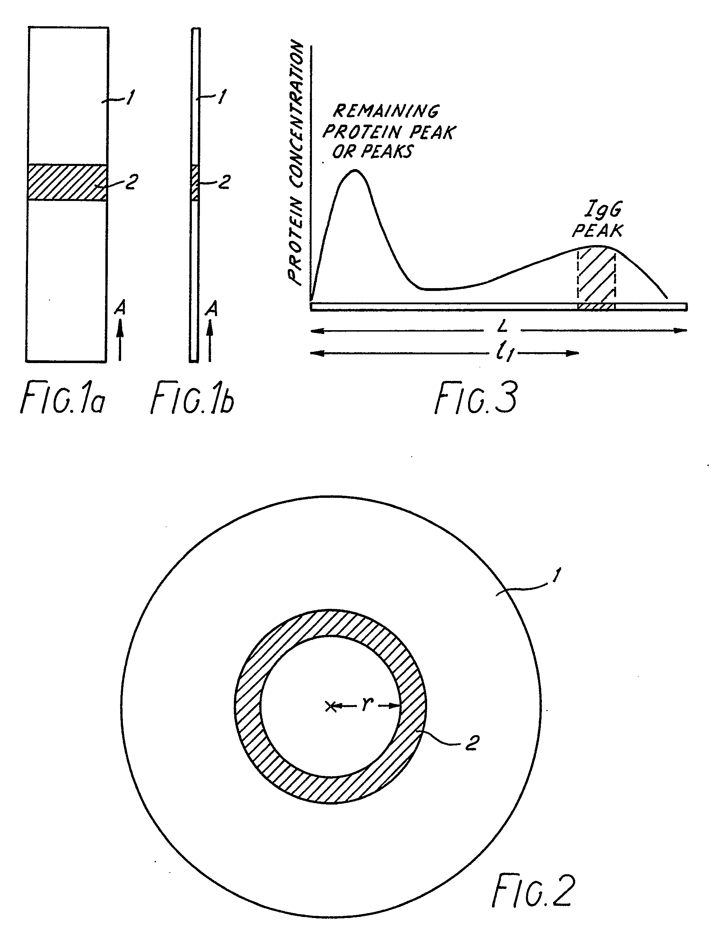

Referring to Figures 1a and 1b, the IgG dipstick comprises a strip 1 of diethylaminoethyl cellulose filter paper impregnated in the region of the band 2 with 3,3,5,5,tetrachloro 3,4,5,6,tetrabromosulphothalein. When the dipstick is dipped into a sample, for example, a blood, milk or colostrum sample in a suitable buffer, e.g. an aqueous tris buffer, pH>8, the sample is drawn up into the dipstick in the direction of the arrow A by capillary attraction. By virtue of its greater rate of diffusion through the ion-exchange paper at its isoelectric point, the IgG contained in the sample will reach and react with the dye in the band 2 ahead of any competing species, which will become concentrated at the tip of the dipstick remote from the band 2. By comparison of the intensity of colouration produced in the band 2, with suitable standard colour charts the concentration of IgG in the original sample can be determined.

In Figure 2, the device is in the form of a circular filter paper 1 of diethylaminoethyl cellulose with a circular band 2 of dye impregnated into the paper about the centre X at a predetermined radius r. Application of a buffered solution of blood, milk or colostrum to the filter paper at the point X will result in radial diffusion of the sample therein towards the band 2 of dye. Once again, by virtue of its greater rate of diffusion through the ion exchange material at its isoelectric point, any IgG in the sample reaches and reacts with the band of dye 2 before the competing protein species.

Referring to Figure 3, this is a graphic representation of the protein concentration gradient obtained in the dipstick 1 after dipping the end A in a buffered solution of the sample, for example, a blood, milk or colostrum sample, the pH of the buffer being adjusted to the isoelectric point of the protein of interest, pH 8 in the case of IgG.

At this pH, the IgG diffuses preferentially through the ion exchange material and since the dipstick is of limited volume, as determined by the length L (and the width and thickness), a protein concentration gradient is established along the length L of the dipstick as indicated, this concentration gradient having two, or possibly more, distinct peaks representing the linear separation of the protein of interest, i.e. the IgG, from the remaining proteins, which are not at their respective isoelectric points and are thus preferentially retained by the ion exchange material. The position of the IgG peak determines the position of the indicator band 2, i.e. the distance $l_1$ from the end of the dipstick. The position of the band 2, i.e. the distance $l_1$, can be determined mathematically from the known diffusion rates of the proteins in ion exchange chromatography procedures, but in practice will usually be determined empirically.

Whilst the invention has been particularly described with reference to the determination of IgG levels in blood, milk or colostrum samples, particularly from cattle, which represent an important veterinary use for the method and device of the present invention, it will be understood that similar techniques can be used for the detection and determination of a variety of other selected proteins in a variety of different samples both from human and animal sources. 7

## Claims

1. A method for the detection and determination of a given protein in a sample containing a mixture of proteins, which comprises allowing the unfractionated sample in admixture with an aqueous buffer solution having a pH value substantially equal to the isoelectric point of the protein of interest, to be drawn by capillary attraction into a porous member of ion exchange material through which the selected protein, being at its isoelectric point, diffuses at a greater rate than other proteins present in the sample and which, by virtue of their differing isoelectric points, are retained by the ion exchange material, the porous ion exchange member being of restricted volume, whereby there is established along the length of the member a concentration gradient of the protein of interest having a maximum at a point intermediate the ends of the member, said maximum being linearly separated from the point of maximum concentration of the other proteins in the sample, said member having an indicator dye incorporated into the ion exchange material at the point of maximum concentration of the protein of interest, and colorimentrically responsive to the concentration of the protein of interest at that point.

2. A method according to claim 1, wherein the protein of interest is an immunoglobulin.

3. A method according to claim 2, wherein the sample is a blood, milk or colostrum sample.

4. A method according to any one of claims 1 to 3, wherein said ion-exchange member is in the form of a dipstick comprising a strip of ion-exchange material impregnated with said indicator dye at a predetermined position intermediate the ends of the strip.

5. A method according to any one of claims 1 to 4, wherein the ion-exchange material is a cellulosic ion-exchange material.

6. A method according to claim 5, wherein the ion-exchange material is diethylaminoethyl cellulose.

7. A device for the detection and determination of a given protein in a sample containing a mixture of proteins, said device comprising a porous ion-exchange member of limited volume into which the sample, in admixture with an aqueous buffer, the pH of which is adjusted to the isoelectric point of the protein of interest, may be drawn by capillary attraction, said ion exchange member having incorporated therein at a predetermined linear distance from the point of application of the sample, an indicator dye colourimetrically reactive with the given protein and capable of reacting therewith upon diffusion of the said protein through the ion-exchange member to produce a band of colouration on said member.

8. A device according to claim 7, in the form of a dipstick comprising said ion-exchange material and which can be dipped into the sample at one end, and having said dye incorporated therein intermediate the ends of the dipstick and at a predetermined distance from said one end.

9. A device according to claim 7 or 8, wherein said ion-exchange member or column comprises a cellulosic ion-exchange material.

10. A device according to claim 9, wherein said ion-exchange material is diethylaminoethyl cellulose.

11. A device according to any one of claims 7 to 10, for the detection and determination of immunoglobulin in blood or colostrum.

12. A device according to claim 7, substantially as hereinbefore described with reference to Figures 1a and 1b, or Figure 2 of the accompanying drawings.

FIG.1a   FIG.1b

FIG.3

PROTEIN CONCENTRATION

REMAINING PROTEIN PEAK OR PEAKS

IgG PEAK

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A-1 377 463 (BOEHRINGER MANNHEIM GmbH)<br>* Claims *<br>--- | 1 | G 01 N 33/68 |
| A | US-A-2 862 796 (E.N. GOMBERG)<br>* Claims *<br>--- | 1 | |
| A | US-A-3 485 587 (A.S. KEATON)<br>* Claims 1,9,10 *<br>----- | 1 | |

**TECHNICAL FIELDS SEARCHED (Int. Cl.4)**

G 01 N

The present search report has been drawn up for all claims

| Place of search | Date of completion of the search | Examiner |
|---|---|---|
| THE HAGUE | 27-12-1988 | MEYLAERTS H. |

EPO FORM 1503 03.82 (P0401)